# EUROPEAN PATENT SPECIFICATION

(11) **EP 1 645 596 B1**
(45) Date of publication and mention of the grant of the patent: **19.09.2018**
(21) Application number: 04747455.6
(22) Date of filing: 07.07.2004
(51) Int. Cl.: C08L 101/14, C08K 3/22, C08K 5/20, A61F 13/53, A61L 15/20, A61L 15/60

(54) **WATER-ABSORBING RESIN COMPOSITION**
WASSERABSORBIERENDE HARZZUSAMMENSETZUNG
COMPOSITION DE RÉSINE ABSORBANT L'EAU

(30) Priority: 11.07.2003 JP 2003273507
(43) Date of publication of application: 12.04.2006
(73) Proprietor: SUMITOMO SEIKA CHEMICALS CO., LTD., Kako-gun, Hyogo 675-0145 (JP)
(72) Inventor: HANDA, Masayoshi, Sumitomo Seika Chem. Co. Ltd, Himeji-shi, Hyogo 672-8076 (JP); TANIGUCHI, Takayasu, Sumitomo Seika Chem. Co. Ltd, Himeji-shi, Hyogo 672-8076 (JP); OIDA, Tatsuya, Sumitomo Seika Chem. Co. Ltd, Himeji-shi, Hyogo 672-8076 (JP); NAWATA, Yasuhiro, Sumitomo Seika Chem. Co. Ltd, Himeji-shi, Hyogo 672-8076 (JP)
(74) Representative: Vossius & Partner Patentanwälte Rechtsanwälte mbB
(86) International application number: PCT/JP2004/009989
(87) International publication number: WO 2005/005549

(56) References cited:
- JP-A- 01 210 463
- JP-A- 08 052 203
- JP-A- 63 118 375
- JP-A- 63 146 964
- JP-A- 63 272 349
- JP-A- 2000 026 738
- JP-A- 2003 206 305
- JP-A- 2003 206 381
- JP-A- 2004 210 924
- US-A- 4 863 989
- US-B2- 6 469 080

## Description

### TECHNICAL FIELD

The present invention relates to a water-absorbent resin composition. More specifically, the present invention relates to a water-absorbent resin composition, and an absorbent and an absorbent article in which the water-absorbent resin composition is used.

### BACKGROUND ART

A water-absorbent article such as disposable diaper or sanitary napkin has been generally formed by interposing an absorbent composed of a hydrophilic fiber and a water-absorbent resin between a liquid-permeable sheet to be placed on the side contacting a body and a liquid-impermeable sheet to be placed on the side opposite to the liquid-permeable sheet.

In recent years, an absorbent, or disposable diaper, sanitary napkin or the like in which the absorbent is used tends to be thinner in order to achieve carrying convenience and a comfortable fit. Along with this thinning, there has been studied the development of an absorbent having a decreased amount of hydrophilic fiber and an increased amount of a water-absorbent resin in order to reduce re-wet or liquid leakage even when the liquid is absorbed in a large amount.

However, since a gel formed by allowing a water-absorbent resin to absorb a body fluid such as human urine generally deteriorates in accordance with the passage of time, its absorbability is lowered. In addition, since the water-absorbent resin itself is partially decomposed as the deterioration of the gel is further progressed, a water-soluble substance is exuded from the gel.

Accordingly, when a manufactured article produced by using this water-absorbent resin is used in contact with a human body, a water-soluble substance exuded from the article is deposited to skin, so that there is a possibility to arise a rash or the like.

Further, in an absorbent containing a water-absorbent resin in a large amount, gel deteriorates in the state where water-absorbent resins are contacted with each other. Therefore, a phenomenon, so-called "gel blocking" in which the exuded water-soluble substance clogs a gap between gel particles to prevent the liquid from being permeated into the water-absorbent resin is likely to take place. This gel blocking is considered to be one of the factors for liquid leakage of the absorbent.

In addition, a water-absorbent resin may discolor into yellow or brown by an external factor such as heat or humidity when the resin is allowed to stand. Particularly, when the water-absorbent resin in the absorbent article such as disposable diapers and sanitary napkins discolors, the external appearance of the absorbent article is impaired, so that its commercial value is drastically lowered.

Therefore, it has been required that the water-absorbent resin is not discolored, even when the water-absorbent resins usable for absorbent articles are stored for a long period of time under severe high-temperature, high-humidity environment such as storehouse in the summertime.

Accordingly, there have been proposed a water-absorbent resin composition containing an oxygen-containing reducing inorganic salt (JP-A-Showa-63-118375), a water-absorbent resin in which tripolyphosphoric acid or a salt thereof is adhered to the water-absorbent resin thereby to be carried thereon (JP-A-Hei-1-33158), and the like in order to improve the stability of a gel. There is, however, a disadvantage in these water-absorbent resins that sufficient discoloration resistance cannot be exhibited.

In addition, in order to improve stability of a gel and discoloration resistance in a water-absorbent resin, there have been proposed a water-absorbent resin composition containing a water-absorbent resin, a reducing compound, an organic carboxylic acid and/or a salt thereof (JP 2003-52742 A), and the like. There is, however, a disadvantage in this composition that sufficient stability of a gel and discoloration resistance cannot be exhibited.

Similarly, US4863989 discloses a water absorbent resin composition comprising a water absorbent resin, at least one oxygen-containing reducing inorganic salt (e.g. sulfites, bisulfites) in an amount of 0.001 to 10 parts by weight and at least one organic antioxidant (e.g. ascorbic acid, gallic acid) in an amount of 0.0001 to 10 parts by weight.

### DISCLOSURE OF INVENTION

An object of the present invention is to provide a water-absorbent resin composition which is excellent in stability of a gel and discoloration resistance.

The present invention relates to:
(1) a water-absorbent resin composition comprising a water-absorbent resin, an oxygen-containing reducing inorganic salt, an aminocarboxylic acid-based metal chelating agent and an organic antioxidant;
(2) an absorbent comprising the water-absorbent resin composition mentioned in the above (1) and a hydrophilic fiber; and
(3) an absorbent article comprising the absorbent mentioned in the above (2) interposed between a liquid-permeable sheet and a liquid-impermeable sheet.

### BEST MODE FOR CARRYING OUT THE INVENTION

One of the great features of the water-absorbent resin composition of the present invention resides in that the water-absorbent resin composition comprises a water-absorbent resin, an oxygen-containing reducing inorganic salt, an aminocarboxylic acid-based metal chelating agent and an organic antioxidant. Since the water-absorbent resin composition of the present invention has this feature, there is exhibited an excellent effect such that the water-absorbent resin composition is excellent in stability of a gel and discoloration resistance.

The water-absorbent resin used in the present invention includes, for instance, crosslinked polymers of acrylic acid salt, crosslinked hydrolysates of starch-acrylic acid salt graftcopolymers, crosslinked copolymers of vinyl alcohol-acrylic acid salt, crosslinked maleic anhydride-grafted polyvinyl alcohol, crosslinked isobutylene-maleic anhydride copolymers, partially neutralized crosslinked polyacrylic acid, saponified vinyl acetate-acrylic ester copolymers, crosslinked polymers of α-hydroxyacrylic acid and the like. Among them, the crosslinked polymers of acrylic acid salt is preferable since the polymer is capable of absorbing water in a large amount and retaining the absorbed water in its molecule even when a certain load is applied to the polymer. The process for preparing such a water-absorbent resin is not limited to specified ones, and includes known processes as described in JP-A-Hei-3-227301 and the like.

The oxygen-containing reducing inorganic salt used in the present invention includes, for instance, sulfites such as sodium sulfite, potassium sulfite, calcium sulfite, zinc sulfite and ammonium sulfite; bisulfites such as sodium bisulfite, potassium bisulfite, calcium bisulfite and ammonium bisulfite; pyrosulfites such as sodium pyrosulfite, potassium pyrosulfite and ammonium pyrosulfite; dithionites such as sodium dithionite, potassium dithionite, ammonium dithionite, calcium dithionite and zinc dithionite; trithionates such as potassium trithionate and sodium trithionate; tetrathionates such as potassium tetrathionate and sodium tetrathionate; thiosulfates such as sodium thiosulfate, potassium thiosulfate and ammonium thiosulfate; nitrites such as sodium nitrite, potassium nitrite, calcium nitrite and zinc nitrite; and the like. Among them, the sulfites, the bisulfites, the pyrosulfites, the dithionites and the nitrites are preferable, the sulfites such as sodium sulfite and potassium sulfite and the bisulfites such as sodium bisulfite and potassium bisulfite are more preferable, from the viewpoint of enhancing stability of a gel and discoloration resistance.

It is desired that the amount of the oxygen-containing reducing inorganic salt is at least 0.01 parts by weight, preferably at least 0.02 parts by weight based on 100 parts by weight of the water-absorbent resin from the viewpoint of enhancing stability of a gel and discoloration resistance. In addition, even when the oxygen-containing reducing inorganic salt is used in an exceedingly large amount, the stability of a gel and discoloration resistance corresponding to the amount are not exhibited, thereby making it uneconomical. Therefore, it is desired that the amount of the oxygen-containing reducing inorganic salt is at most 5 parts by weight, preferably at most 3 parts by weight based on 100 parts by weight of the water-absorbent resin. Accordingly, it is desired that the amount of the oxygen-containing reducing inorganic salt is 0.01 to 5 parts by weight, preferably 0.02 to 3 parts by weight based on 100 parts by weight of the water-absorbent resin in view of these viewpoints.

The aminocarboxylic acid-based metal chelating agent used in the present invention includes, for instance, iminodiacetic acid, hydroxyethyliminodiacetic acid, nitrilotriacetic acid, nitrilotripropionic acid, ethylenediaminetetraacetic acid, hydroxyethylenediaminetriacetic acid, hexamethylenediaminetetraacetic acid, diethylenetriaminepentaacetic acid, triethylenetetraminehexaacetic acid, trans-1,2-diaminocyclohexanetetraacetic acid, bis(2-hydroxyethyl)glycine, diaminopropanoltetraacetic acid, ethylenediamine-2-propionic acid, glycol ether diaminetetraacetic acid, bis(2-hydroxybenzyl)ethylenediaminediacetic acid, and salts thereof, and the like. Among them, ethylenediaminetetraacetic acid, hydroxyethylenediaminetriacetic acid, hexamethylenediaminetetraacetic acid, diethylenetriaminepentaacetic acid, triethylenetetraminehexaacetic acid, trans-1,2-diaminocyclohexanetetraacetic acid, diaminopropanoltetraacetic acid, glycol ether diaminetetraacetic acid, and salts thereof are preferable, and ethylenediaminetetraacetic acid, hydroxyethylenediaminetriacetic acid, diethylenetriaminepentaacetic acid, triethylenetetraminehexaacetic acid, trans-1,2-diaminocyclohexanetetraacetic acid, and salts thereof are more preferable, from the viewpoint of having an excellent effect of preventing discoloration.

It is desired that the amount of the aminocarboxylic acid-based metal chelating agent is at least 0.01 parts by weight, preferably at least 0.05 parts by weight, more preferably at least 0.1 parts by weight based on 100 parts by weight of the water-absorbent resin from the viewpoint of exhibiting sufficient effect of preventing discoloration. In addition, even when the aminocarboxylic acid-based metal chelating agent is used in an exceedingly large amount, the effect of preventing discoloration corresponding to the amount is not exhibited, thereby making it uneconomical. Therefore, it is desired that the amount of the aminocarboxylic acid-based metal chelating agent is at most 5 parts by weight, preferably at most 3 parts by weight, more preferably at most 2 parts by weight based on 100 parts by weight of the water-absorbent resin. Accordingly, it is desired that the amount of the aminocarboxylic acid-based metal chelating agent is 0.01 to 5 parts by weight, preferably 0.05 to 3 parts by weight, more preferably 0.1 to 2 parts by weight based on 100 parts by weight of the water-absorbent resin in view of these viewpoints.

The organic antioxidant used in the present invention is not limited to specified ones, and includes ascorbic acids such as L-ascorbic acid, sodium L-ascorbate, D-ascorbic acid and sodium D-ascorbate; erythorbic acids such as erythorbic acid and sodium erythorbate; gallic acids such as gallic acid, methyl gallate, ethyl gallate, n-propyl gallate, isoamyl gallate, octyl gallate and lauryl gallate; protocatechuic acids such as protocatechuic acid and ethyl protocatechuate; benzimidazoles such as 2-mercaptobenzimidazole; and alkylated hydroxyanisoles such as butylated hydroxyanisole. Among them, the ascorbic acids, the erythorbic acids and the gallic acids are preferable, and L-ascorbic acid, sodium L-ascorbate, sodium erythorbate and n-propyl gallate are more preferable, because these compounds make a gel to be excellent in stability to an electrolytic aqueous solution.

It is desired that the amount of the organic antioxidant is at least 0.001 parts by weight, preferably at least 0.005 parts by weight based on 100 parts by weight of the water-absorbent resin from the viewpoint of exhibiting sufficient stability of the gel to the electrolytic aqueous solution. In addition, even when the organic antioxidant is used in an exceedingly large amount, the stability of a gel corresponding to the amount is not exhibited, thereby making it uneconomical. Therefore, it is desired that the amount of the organic antioxidant is at most 5 parts by weight, preferably at most 2 parts by weight based on 100 parts by weight of the water-absorbent resin. Accordingly, it is desired that the amount of the organic antioxidant is 0.001 to 5 parts by weight, preferably 0.005 to 2 parts by weight based on 100 parts by weight of the water-absorbent resin in view of these viewpoints.

The water-absorbent resin composition of the present invention can be obtained by, for instance, mixing a water-absorbent resin, an oxygen-containing reducing inorganic salt, an aminocarboxylic acid-based metal chelating agent and an organic antioxidant. The process for mixing these components and the order of adding these components are not limited to specified ones.

The process for mixing a water-absorbent resin with an oxygen-containing reducing inorganic salt, an aminocarboxylic acid-based metal chelating agent and an organic antioxidant include, for instance, (i) a process comprising adding an oxygen-containing reducing inorganic salt, an aminocarboxylic acid-based metal chelating agent and an organic antioxidant to an aqueous solution of a monomer before polymerization, which constitutes a water-absorbent resin, and mixing them; (ii) a process comprising adding an oxygen-containing reducing inorganic salt, an aminocarboxylic acid-based metal chelating agent and an organic antioxidant to a water-containing gel-like product of a water-absorbent resin, and mixing them; (iii) a process comprising adding an oxygen-containing reducing inorganic salt, an aminocarboxylic acid-based metal chelating agent and an organic antioxidant to a water-absorbent resin after drying the water-absorbent resin, and mixing them; (iv) a process comprising adding an oxygen-containing reducing inorganic salt, an aminocarboxylic acid-based metal chelating agent and an organic antioxidant to a water-absorbent resin after dispersing the water-absorbent resin in an organic solvent, and heating and removing the solvent from the mixture; and the like. Among these processes, the above-mentioned process (iii) is preferable since the procedures are simple and convenient.

In the process comprising adding an oxygen-containing reducing inorganic salt, an aminocarboxylic acid-based metal chelating agent and an organic antioxidant to a water-absorbent resin after drying the water-absorbent resin, and mixing them, the aminocarboxylic acid-based metal chelating agent contains particles having a particle diameter of at most 106 µm in a content of preferably at least 50% by weight, more preferably at least 80% by weight, from the viewpoint of exhibiting a sufficient effect of preventing discoloration.

The mixer used in adding an oxygen-containing reducing inorganic salt, an aminocarboxylic acid-based metal chelating agent and an organic antioxidant to a water-absorbent resin after drying the water-absorbent resin includes, for instance, a Nauta mixer, a Ribbon blender, a cross-rotary mixer, a conical blender, a double-arm type kneader, a screw-type blender, a V-shaped blender, a W-shaped blender, a turbulizer, a Mechanomill and the like. The present invention, however, is not limited only to those exemplified ones.

In the present invention, an absorbent can be obtained by using the above-mentioned water-absorbent resin composition. The absorbent comprises a water-absorbent resin composition and a hydrophilic fiber.

The hydrophilic fiber includes, for instance, a cellulose fiber, an artificial cellulose fiber, and the like. The hydrophilic fiber may contain a synthetic fiber having hydrophobicity within the range that does not hinder the object of the present invention.

The content of the water-absorbent resin composition in the absorbent is preferably at least 45% by weight, more preferably at least 55% by weight from the viewpoint of sufficiently absorbing a body fluid such as urine and giving a comfortable fit. In addition, the content of the water-absorbent resin composition in the absorbent is preferably at most 95% by weight, more preferably 90% by weight in view of containing the hydrophilic fiber and the like therein in order to enhance the retention of the shape of the resulting absorbent.

Preferred embodiments of the absorbent include, for instance, a mixed-type dispersion obtained by mixing a water-absorbent resin composition with a hydrophilic fiber so as to have a uniform composition; a sandwiched-type structure in which a water-absorbent resin composition is interposed between two layers of hydrophilic fibers.

An absorbent article can be produced, for instance, by interposing the above-mentioned absorbent between a liquid-permeable sheet and a liquid-impermeable sheet.

The liquid-permeable sheet includes, for instance, air-through type nonwoven fabrics, spun bond type nonwoven fabrics, chemical bond type nonwoven fabrics, needle-punched type nonwoven fabrics, and the like, which are composed of fibers of polyethylene, polypropylene, polyester or the like.

The liquid-impermeable sheet includes, for instance, synthetic resin films made of a resin such as polyethylene, polypropylene or polyvinyl chloride, and the like.

The kind of the absorbent article is not limited to specified ones. Representative examples of the absorbent article include hygienic materials such as disposable diaper, sanitary napkin and incontinence pad; urine-absorbing materials for pets; materials for civil engineering and construction such as packing materials; food freshness retaining materials such as drip absorbents and cold-reserving agents; horticultural articles such as water-retaining materials for soils; and the like.

### EXAMPLES

The present invention will be described more specifically hereinbelow by means of Examples and Comparative Examples, without intending to limit the present invention only to these Examples.

### Preparation Example

Five-hundred milliliters of n-heptane was added to a 1000 mL-five-neck cylindrical round bottomed flask equipped with a stirrer, a reflux condenser, a dropping funnel, a thermometer and a nitrogen gas inlet tube. Thereto was added 0.92 g of a sucrose fatty acid ester to disperse. The temperature of the mixture was raised to dissolve the ester, and thereafter cooled to 55°C.

Separately from the above, 92 g of an 80% by weight aqueous acrylic acid solution was added to a 500 mL-Erlenmeyer flask. The amount 102.2 g of a 30% by weight aqueous sodium hydroxide solution was added dropwise to this Erlenmeyer flask with externally cooling the mixture to neutralize 75% by mol of acrylic acid, thereby giving a partially neutralized product of acrylic acid. Further, 50.2 g of water, 0.11 g of potassium persulfate as a polymerization initiator, and 18.4 mg of ethylene glycol diglycidyl ether as a crosslinking agent were added thereto to give an aqueous monomer solution for a first step polymerization.

The entire amount of this aqueous monomer solution for a first step polymerization was added to the above-mentioned five-neck cylindrical round bottomed flask with stirring to disperse. The internal of the system was sufficiently replaced with nitrogen gas, and thereafter the temperature of the mixture was raised. The polymerization reaction was carried out for 1 hour with keeping the bath temperature at 70°C. Thereafter, the mixture was cooled to room temperature to give a polymerization slurry.

The amount 119.1 g of an 80% by weight aqueous acrylic acid solution was added to another 500 mL-Erlenmeyer flask. Thereto was added dropwise 132.2 g of a 30% by weight aqueous sodium hydroxide solution with cooling the mixture, to neutralize 75% by mol of acrylic acid. The amount 27.4 g of water, 0.14 g of potassium persulfate, and 35.7 mg of ethylene glycol diglycidyl ether were added thereto to give an aqueous monomer solution for a second step polymerization. The aqueous solution was cooled in an ice water bath.

The entire amount of this aqueous monomer solution for a second step polymerization was added to the polymerization slurry obtained above. Thereafter, the internal of the system was again sufficiently replaced with nitrogen, the temperature of the mixture was then raised, and the second-step polymerization reaction was carried out for 2 hours with keeping the bath temperature at 70°C. After the termination of the polymerization reaction, only water was removed from the water-containing gel-like product being dispersed in n-heptane to the external of the system by azeotropic distillation. To the gel-like product obtained was added 8.44 g of a 2% by weight aqueous solution of ethylene glycol diglycidyl ether, and the mixture was dried by further removing water and n-heptane from the mixture by distillation to give 214.4 g of a water-absorbent resin.

### Example 1

A 2 L-polyethylene container was charged with 100 g of the water-absorbent resin obtained in Preparation Example, 1 g of sodium sulfite, 0.5 g of disodium ethylenediaminetetraacetate (content of particles having a particle diameter of at most 106 µm: 86% by weight) and 0.05 g of L-ascorbic acid. The ingredients were mixed for 1 hour using a cross-rotary mixer (manufactured by MEIWA KOGYO CO., LTD., product number: CM-3) at an autorotation speed of 30 rpm and a revolution speed of 30 rpm, to give 101.5 g of a water-absorbent resin composition.

### Example 2

A 2 L-polyethylene container was charged with 100 g of a water-absorbent resin obtained in the same manner as in Preparation Example, 2 g of sodium sulfite, 0.7 g of diethylenetriaminepentaacetic acid (content of particles having a particle diameter of at most 106 µm: 87% by weight) and 0.02 g of L-ascorbic acid. The ingredients were mixed for 1 hour using a cross-rotary mixer (manufactured by MEIWA KOGYO CO., LTD., product number: CM-3) at an autorotation speed of 30 rpm and a revolution speed of 30 rpm, to give 102.7 g of a water-absorbent resin composition.

### Example 3

A 2 L-polyethylene container was charged with 100 g of a water-absorbent resin obtained in the same manner as in Preparation Example, 2 g of sodium bisulfite, 0.8 g of ethylenediaminetetraacetic acid (content of particles having a particle diameter of at most 106 µm: 88% by weight) and 0.1 g of sodium erythorbate. The ingredients were mixed for 1 hour using a cross-rotary mixer (manufactured by MEIWA KOGYO CO., LTD., product number: CM-3) at an autorotation speed of 30 rpm and a revolution speed of 30 rpm, to give 102.9 g of a water-absorbent resin composition.

### Example 4

A 2 L-polyethylene container was charged with 100 g of a water-absorbent resin obtained in the same manner as in Preparation Example, 2.5 g of potassium pyrosulfite, 1.2 g of triethylenetetraminehexaacetic acid (content of particles having a particle diameter of at most 106 µm: 90% by weight) and 0.1 g of n-propyl gallate. The ingredients were mixed for 1 hour using a cross-rotary mixer (manufactured by MEIWA KOGYO CO., LTD., product number: CM-3) at an autorotation speed of 30 rpm and a revolution speed of 30 rpm, to give 103.8 g of a water-absorbent resin composition.

### Example 5

A 2 L-polyethylene container was charged with 100 g of a water-absorbent resin obtained in the same manner as in Preparation Example, 2.5 g of sodium dithionite, 1 g of trans-1,2-diaminocyclohexanetetraacetic acid (content of particles having a particle diameter of at most 106 µm: 86% by weight) and 0.5 g of ethyl protocatechuate. The ingredients were mixed for 1 hour using a cross-rotary mixer (manufactured by MEIWA KOGYO CO., LTD., product number: CM-3) at an autorotation speed of 30 rpm and a revolution speed of 30 rpm, to give 104 g of a water-absorbent resin composition.

### Example 6

A 2 L-polyethylene container was charged with 100 g of a water-absorbent resin obtained in the same manner as in Preparation Example, 3 g of sodium nitrite, 1.5 g of trisodium hydroxyethylenediaminetriacetate (content of particles having a particle diameter of at most 106 µm: 89% by weight) and 1 g of butylated hydroxyanisole. The ingredients were mixed for 1 hour using a cross-rotary mixer (manufactured by MEIWA KOGYO CO., LTD., product number: CM-3) at an autorotation speed of 30 rpm and a revolution speed of 30 rpm, to give 105.5 g of a water-absorbent resin composition.

### Example 7

A 2 L-polyethylene container was charged with 100 g of a water-absorbent resin obtained in the same manner as in Preparation Example, 1 g of sodium sulfite, 0.5 g of disodium ethylenediaminetetraacetate (content of particles having a particle diameter of at most 106 µm: 36% by weight) and 0.05 g of L-ascorbic acid. The ingredients were mixed for 1 hour using a cross-rotary mixer (manufactured by MEIWA KOGYO CO., LTD., product number: CM-3) at an autorotation speed of 30 rpm and a revolution speed of 30 rpm, to give 101.5 g of a water-absorbent resin composition.

### Comparative Example 1

The water-absorbent resin obtained in Preparation Example was directly used.

### Comparative Example 2

The same procedures as in Example 1 were carried out except that 1 g of sodium sulfite in Example 1 was not used, to give 100.6 g of a water-absorbent resin composition.

### Comparative Example 3

The same procedures as in Example 1 were carried out except that 0.5 g of disodium ethylenediaminetetraacetate in Example 1 was not used, to give 101.1 g of a water-absorbent resin composition.

### Comparative Example 4

The same procedures as in Example 1 were carried out except that 0.05 g of L-ascorbic acid in Example 1 was not used, to give 101.5 g of a water-absorbent resin composition.

### Evaluation

Physical properties of a water-absorbent resin composition and a water-absorbent resin, which were obtained in each Example and each Comparative Example, and physical properties of absorbent articles obtained by using them, were evaluated in accordance with the following methods.

### (1) Stability of Gel During Absorption of Human Urine

Thirty-nine grams of human urine taken from adult male was added to a 100 mL-beaker, and 1 g of a water-absorbent resin composition or a water-absorbent resin was added thereto to prepare a human urine-absorbed gel. This human urine-absorbed gel was allowed to stand for 24 hours in an incubator at 40°C. Thereafter, stability of the gel was evaluated in accordance with the following evaluation criteria.

### [Evaluation Criteria]

| | |
|---|---|
| ⊚: | Gel has elasticity and is not crushed even when strongly pressed. |
| ○: | Gel has elasticity but is crushed when strongly pressed. |
| Δ: | Gel maintains its shape but is crushed when lightly pinched between fingers. |
| ×: | Shape of the gel has collapsed. |

### (2) Stability of Gel During Absorption of Physiological Saline

Thirty-nine grams of a 0.9% by weight physiological saline was added in a 100 mL-beaker, and 1 g of a water-absorbent resin composition or a water-absorbent resin was added thereto to prepare a physiological saline-absorbed gel. This physiological saline-absorbed gel was allowed to stand for 24 hours in an incubator at 40°C. Thereafter, stability of the gel was evaluated in accordance with the following evaluation criteria.

### [Evaluation Criteria]

| | |
|---|---|
| ⊚: | Gel has elasticity and is not crushed even when strongly pressed. |
| ○: | Gel has elasticity but is crushed when strongly pressed. |
| Δ: | Gel maintains its shape but is crushed when lightly pinched between fingers. |
| ×: | Shape of the gel has collapsed. |

### (3) Discoloration Resistance of Absorbent Article

Twelve grams of a water-absorbent resin composition or a water-absorbent resin and 9 g of disintergrated wooden pulp were dry-blended. The resultant mixture was sprayed over a piece of tissue paper having a size of 40 cm x 10 cm and a weight of 1 g, and thereafter another piece of tissue paper of the same weight and size was superposed. A load of 196 kPa was applied entirely to the resulting sheet for 30 seconds to press the sheet, thereby to prepare an absorbent. This absorbent was interposed between a polyethylene air-through-type nonwoven fabric having a size of 40 cm x 12 cm and a basis weight of 20 g/m² and a polyethylene sheet of the same size having a weight of 1 g, to prepare an absorbent article.

The resulting absorbent article was allowed to stand for 10 days in a thermohygrostat set at a temperature of 50° ± 2°C and a relative humidity of 90 ± 2%. Thereafter, discoloration of the water-absorbent resin composition or the water-absorbent resin in the absorbent article was visually observed, and discoloration resistance of the absorbent article was evaluated on the basis of the following evaluation criteria.

### [Evaluation Criteria]

A: The internal water-absorbent resin is not discolored when observed after the nonwoven fabric is removed and the absorbent is loosened.
B: Discoloration of the water-absorbent resin is not found when observed without removing the nonwoven fabric, but discoloration is found in a part of the water-absorbent resin when the nonwoven fabric is removed and the absorbent is loosened.
C: Discoloration of the water-absorbent resin is found even when observed without removing the nonwoven fabric.

The kind and the amount added (parts by weight) of the oxygen-containing reducing inorganic salt, the aminocarboxylic acid-based metal chelating agent and the organic antioxidant used in each Example and each Comparative Example are shown in Table 1.

**Table 1**

| Ex. No. | Oxygen-containing Reducing Inorganic Salt | | Aminocarboxylic Acid-based Metal Chelating Agent | | Organic Antioxidant | |
|---|---|---|---|---|---|---|
| | Kind | Amount Added (parts by weight) | Kind | Amount Added (parts by weight) | Kind | Amount Added (parts by weight) |
| 1 | Sodium Sulfite | 1 | Disodium Ethylenediaminetetraacetate | 0.5 | L-ascorbic acid | 0.05 |
| 2 | Sodium Sulfite | 2 | Diethylenetriaminepentaacetic Acid | 0.7 | L-ascorbic acid | 0.02 |
| 3 | Sodium Bisulfite | 2 | Ethylenediaminetetraacetic Acid | 0.8 | Sodium Erythorbate | 0.1 |
| 4 | Potassium Pyrosulfite | 2.5 | Triethylenetetraminehexaacetic Acid | 1.2 | n-Propyl Gallate | 0.1 |
| 5 | Sodium Dithionite | 2.5 | Trans-1,2-diaminocyclohexanetetraacetic Acid | 1 | Ethyl Protocatechuate | 0.5 |
| 6 | Sodium Nitrite | 3 | Trisodium hydroxyethylenediaminetriacetate | 1.5 | Butylated Hydroxyanisole | 1 |
| 7 | Sodium Sulfite | 1 | Disodium Ethylenediaminetetraacetate | 0.5 | L-ascorbic acid | 0.05 |

| Comp. Ex. | | | | | | |
|---|---|---|---|---|---|---|
| 1 | (Not Added) | | (Not Added) | | (Not Added) | |
| 2 | (Not Added) | | Disodium Ethylenediaminetetraacetate | 0.5 | L-ascorbic acid | 0.05 |
| 3 | Sodium Sulfite | 1 | (Not Added) | | L-ascorbic acid | 0.05 |
| 4 | Sodium Sulfite | 1 | Disodium Ethylenediaminetetraacetate | 0.5 | (Not Added) | |

As the physical properties of the water-absorbent resin composition and the water-absorbent resin obtained in each Example and each Comparative Example, (1) stability of gel during absorption of human urine and (2) stability of gel during absorption of physiological saline were evaluated in accordance with the above-mentioned methods. In addition, (3) discoloration resistance of absorbent article described above was evaluated using the absorbent article for discoloration resistance test, as prepared using the water-absorbent resin composition or the water-absorbent resin obtained. The results are shown in Table 2.

**Table 2**

| | Stability of Gel During | Stability of Gel During | Discoloration Resistance of Absorbent Article |
|---|---|---|---|
| Ex. No. | Absorption of Human Urine | Absorption of Physiological Saline | |
| 1 | ⊚ | ⊚ | A |
| 2 | ⊚ | ⊚ | A |
| 3 | ⊚ | ⊚ | A |
| 4 | ⊚ | ⊚ | A |
| 5 | ⊚ | ⊚ | A |
| 6 | ⊚ | ⊚ | A |
| 7 | ⊚ | ⊚ | B |

| Comp. Ex. | | | |
|---|---|---|---|
| 1 | × | ○ | B |
| 2 | × | × | A |
| 3 | ⊚ | ⊚ | C |
| 4 | ⊚ | × | A |

It can be seen from the results shown in Table 2 that according to each Example, there is obtained a water-absorbent resin composition which is excellent in stability of a gel, and excellent in discoloration resistance even at high temperatures and high humidity. It can be also seen that the absorbent articles of Examples 1 to 6 in which an aminocarboxylic acid-based metal chelating agent having a content of particles having a particle diameter of at most 106 µm of at least 80% by weight, which is higher than that in Example 7, is used are more excellent in discoloration resistance than the absorbent article of Example 7 in which an aminocarboxylic acid-based metal chelating agent having a content of particles having a particle diameter of at most 106 µm of 36% by weight is used.

As explained above, the water-absorbent resin composition of the present invention is excellent in stability of a gel and discoloration resistance. Therefore, when the water-absorbent resin composition is used, an absorbent and an absorbent article being excellent in these physical properties can be obtained.

### INDUSTRIAL APPLICABILITY

The water-absorbent resin composition of the present invention can be used in absorbent articles such as disposable diaper, sanitary napkin, and the like.

## Claims

1. A water-absorbent resin composition comprising a water-absorbent resin, an oxygen-containing reducing inorganic salt, an aminocarboxylic acid-based metal chelating agent and an organic antioxidant.

2. The water-absorbent resin composition according to claim 1, wherein the amount of the oxygen-containing reducing inorganic salt is 0.01 to 5 parts by weight based on 100 parts by weight of the water-absorbent resin.

3. The water-absorbent resin composition according to claim 1 or 2, wherein the amount of the aminocarboxylic acid-based metal chelating agent is 0.01 to 5 parts by weight based on 100 parts by weight of the water-absorbent resin.

4. The water-absorbent resin composition according to any one of claims 1 to 3, wherein the amount of the organic antioxidant is 0.001 to 5 parts by weight based on 100 parts by weight of the water-absorbent resin.

5. The water-absorbent resin composition according to any one of claims 1 to 4, wherein the oxygen-containing reducing inorganic salt is at least one member selected from the group consisting of sulfites, bisulfites, pyrosulfites, dithionites and nitrites.

6. The water-absorbent resin composition according to any one of claims 1 to 5, wherein the aminocarboxylic acid-based metal chelating agent is at least one member selected from the group consisting of ethylenediaminetetraacetic acid, hydroxyethylenediaminetriacetic acid, diethylenetriaminepentaacetic acid, triethylenetetraminehexaacetic acid, trans-1,2-diaminocyclohexanetetraacetic acid, and salts thereof.

7. The water-absorbent resin composition according to any one of claims 1 to 6, wherein the organic antioxidant is at least one member selected from the group consisting of ascorbic acids, erythorbic acids, gallic acids, protocatechuic acids, benzimidazoles and alkylated hydroxyanisoles.

8. An absorbent comprising the water-absorbent resin composition as defined in any one of claims 1 to 7 and a hydrophilic fiber.

9. An absorbent article comprising the absorbent as defined in claim 8 interposed between a liquid-permeable sheet and a liquid-impermeable sheet.

## Patentansprüche

1. Eine wasserabsorbierende Harzzusammensetzung, umfassend ein wasserabsorbierendes Harz, ein Sauerstoff enthaltendes, reduzierendes anorganisches Salz, ein Metall chelatierendes Mittel auf Aminocarbonsäure-Basis und ein organisches Antioxidans.

2. Die wasserabsorbierende Harzzusammensetzung nach Anspruch 1, wobei die Menge des Sauerstoff enthaltenden, reduzierenden anorganischen Salzes 0,01 bis 5 Gewichtsteile, bezogen auf 100 Gewichtsteile des wasserabsorbierenden Harzes, beträgt.

3. Die wasserabsorbierende Harzzusammensetzung nach Anspruch 1 oder 2, wobei die Menge des Metall chelatierenden Mittels auf Aminocarbonsäure-Basis 0,01 bis 5 Gewichtsteile, bezogen auf 100 Gewichtsteile des wasserabsorbierenden Harzes, beträgt.

4. Die wasserabsorbierende Harzzusammensetzung nach einem der Ansprüche 1 bis 3, wobei die Menge des organischen Antioxidans 0,001 bis 5 Gewichtsteile, bezogen auf 100 Gewichtsteile des wasserabsorbierenden Harzes, beträgt.

5. Die wasserabsorbierende Harzzusammensetzung nach einem der Ansprüche 1 bis 4, wobei das Sauerstoff enthaltende, reduzierende anorganische Salz mindestens ein Mitglied, ausgewählt aus der Gruppe bestehend aus Sulfiten, Bisulfiten, Pyrosulfiten, Dithioniten und Nitriten, ist.

6. Die wasserabsorbierende Harzzusammensetzung nach einem der Ansprüche 1 bis 5, wobei ein Metall chelatierendes Mittel auf Aminocarbonsäure-Basis mindestens ein Mitglied, ausgewählt aus der Gruppe bestehend aus Ethylendiamintetraessigsäure, Hydroxyethylendiamintriessigsäure, Diethylentriaminpentaessigsäure, Triethylentetraminhexaessigsäure, trans-1,2-Diaminocyclohexantetraessigsäure und Salzen davon, ist.

7. Die wasserabsorbierende Harzzusammensetzung nach einem der Ansprüche 1 bis 6, wobei das organische Antioxidans mindestens ein Mitglied, ausgewählt aus der Gruppe bestehend aus Ascorbinsäuren, Erythorbinsäuren, Gallussäuren, Protocatechusäuren, Benzimidazolen und alkylierten Hydroxyanisolen, ist.

8. Ein Absorptionsmittel, umfassend die wasserabsorbierende Harzzusammensetzung, wie in einem der Ansprüche 1 bis 7 definiert, und eine hydrophile Faser.

9. Ein absorbierender Gegenstand, umfassend das Absorptionsmittel, wie in Anspruch 8 definiert, angeordnet zwischen einer flüssigkeitsdurchlässigen Lage und einer flüssigkeitsundurchlässigen Lage.

## Revendications

1. Composition de résine absorbant l'eau comprenant une résine absorbant l'eau, un sel inorganique réducteur contenant de l'oxygène, un agent chélateur de métaux à base d'acide aminocarboxylique et un antioxydant organique.

2. Composition de résine absorbant l'eau selon la revendication 1, dans laquelle la quantité de sel inorganique réducteur contenant de l'oxygène est de 0,01 à 5 parties en poids pour 100 parties en poids de la résine absorbant l'eau.

3. Composition de résine absorbant l'eau selon la revendication 1 ou 2, dans laquelle la quantité d'agent chélateur de métaux à base d'acide aminocarboxylique est de 0,01 à 5 parties en poids pour 100 parties en poids de la résine absorbant l'eau.

4. Composition de résine absorbant l'eau selon l'une quelconque des revendications 1 à 3, dans laquelle la quantité d'antioxydant organique est de 0,001 à 5 parties en poids pour 100 parties en poids de la résine absorbant l'eau.

5. Composition de résine absorbant l'eau selon l'une quelconque des revendications 1 à 4, dans laquelle le sel inorganique réducteur contenant de l'oxygène est au moins un membre choisi dans le groupe constitué par les sulfites, les bisulfites, les pyrosulfites, les dithionites et les nitrites.

6. Composition de résine absorbant l'eau selon l'une quelconque des revendications 1 à 5, dans laquelle l'agent chélateur de métaux à base d'acide aminocarboxylique est au moins un membre choisi dans le groupe constitué par l'acide éthylènediaminetétraacétique, l'acide hydroxyéthylènediaminetriacétique, l'acide diéthylènetriaminepentaacétique, l'acide triéthylènetétraminehexatriacétique, l'acide trans-1,2-diaminocyclohexanetétraacétique, et leurs sels.

7. Composition de résine absorbant l'eau selon l'une quelconque des revendications 1 à 6, dans laquelle l'antioxydant organique est au moins un membre choisi dans le groupe constitué par les acides ascorbiques, les acides érythorbiques, les acides galliques, les acides protocatéchuiques, les benzimidazoles et les hydroxyanisoles alkylés.

8. Absorbant comprenant la composition de résine absorbant l'eau selon l'une quelconque des revendications 1 à 7 et une fibre hydrophile.

9. Article absorbant comprenant l'absorbant selon la revendication 8 interposé entre une feuille perméable aux liquides et une feuille imperméable aux liquides.
